# EUROPÄISCHE PATENTSCHRIFT

(11) **EP 1 809 540 B1**
(45) Veröffentlichungstag und Bekanntmachung des Hinweises auf die Patenterteilung: **24.11.2010**
(21) Anmeldenummer: 05797226.7
(22) Anmeldetag: 14.10.2005
(51) Int. Cl.: B65D 1/09, A61J 1/06

(54) **BEHÄLTER, INSBESONDERE AMPULLE**
CONTAINER, IN PARTICULAR AN AMPOULE
RECIPIENT, EN PARTICULIER AMPOULE

(30) Priorität: 08.11.2004 DE 102004054151; 08.11.2004 DE 202004017346 U
(43) Veröffentlichungstag der Anmeldung: 25.07.2007
(73) Patentinhaber: Hansen, Bernd, 74429 Sulzbach-Laufen (DE)
(72) Erfinder: Hansen, Bernd, 74429 Sulzbach-Laufen (DE)
(74) Vertreter: Bartels, Martin Erich Arthur
(86) Internationale Anmeldenummer: PCT/EP2005/011057
(87) Internationale Veröffentlichungsnummer: WO 2006/048105

(56) Entgegenhaltungen:
- EP-A- 0 930 238
- DE-A1- 3 525 098
- US-A1- 2003 140 921
- US-A1- 2004 139 968

## Beschreibung

Die Erfindung betrifft einen Behälter, insbesondere Ampulle, gemäß der Merkmalsausgestaltung des Oberbegriffes des Patentanspruches 1.

Durch die DE 38 33 036 C2 ist ein Doppelkammerbehälter bekannt, insbesondere Doppelkammerampulle, dessen beide Kammern durch zwei parallel nebeneinander angeordnete Behältnisse gebildet sind, die entfernbare Verschlüsse aufweisen, welche an einen einzigen Knebel angeformt sind. Bei der bekannten Lösung sind die beiden Behältnisse lösbar miteinander verbunden, wobei das die Füll- und/oder Entnahmeöffnung bildende Halsteil des einen Behältnisses mit einer sich zum freien Ende dieses Halsteiles hin verjüngenden konischen Aussenmantelfläche versehen ist und wobei das die Füll- und/oder Entnahmeöffnung bildende Halsteil des anderen Behältnisses mit einem Innenkonus versehen ist, der korrespondierend zu dem Aussenkonus des anderen Behältnisses ausgebildet ist und sich gegen das Innere des Behältnisses hin verjüngt.

Mit der bekannten Doppelkammerlösung können die Inhalte der beiden Behältnisse zusammengebracht und miteinander vermischt werden, ohne dass hierzu ein zusätzliches Behältnis erforderlich wäre. Der dichte Steckverschluss über die Konen erlaubt es bei der bekannten Lösung auch die beiden Behältnisse zu schütteln. Dadurch, dass die beiden Verschlüsse an einem einzigen Knebel angeformt sind, können sie praktisch gleichzeitig mit Hilfe dieses Knebels von dem sie tragenden Halsteil entfernt und ein Mischvorgang eingeleitet werden. Sofern bei der bekannten Lösung ferner wenigstens eines der beiden Behältnisse deformierbar ist, kann es vor dem Herstellen der Steckverbindung etwas zusammengedrückt werden, wodurch ein leichter Unterdruck entsteht, durch den eine Flüssigkeit (Abgabemedium) aus dem anderen Behältnis herausgesaugt werden kann. Weiterhin ist sichergestellt, dass das Halsteil des einen Behältnisses einen Innenkonus bildet, der bei Ampullen zweckmäßigerweise entsprechend den Normen für medizinische Geräte ausgebildet ist, sodass in das Halsteil der Kegel einer Spritze eingesetzt werden kann. Ferner besteht die Möglichkeit, das eine Behältnis nicht zu füllen. Im zusammengesteckten Zustand steht dann ein relativ großer Raum zur Verfügung. Dies kann beispielsweise dann von Vorteil sein, wenn der Inhalt des befüllten Behältnisses vor Gebrauch hin- und herbewegt oder stark geschüttelt werden muß, beispielsweise um seine Applizierung erst zu ermöglichen.

Nachteilig bei der bekannten Lösung ist, dass jeder Behälter immer nur eine spezielle Art an Behälteröffnung aufweist, sodass immer nur Paare an Behältern mit ihren einander zugehörigen korrespondierenden Abgabeöffnungen dergestalt miteinander verbindbar sind. Da solche Behälter regelmäßig in Mehrfachnebeneinanderanordnung in Ampullenblöcken zusammengefaßt sind, können sich dann Probleme ergeben, wenn man die zugehörigen Paare, insbesondere nach deren Trennung vom Ampullenblock erst passend zusammensuchen muß. Werden die Behälter einzeln benutzt, ist darüber hinaus nicht auszuschliessen, dass gerade für die vorgesehene Anschlussmöglichkeit in Form der Abgabeöffnungen an der jeweiligen Abgabekammer gerade kein passender Füll- und/oder Entnahmeanschluss vor Ort unmittelbar vorhanden ist. Ferner sind bei der bekannten Lösung die Herstellkosten erhöht, da man die Werkzeuge für zwei unterschiedliche Arten an Behältertypen zur Verfügung zu stellen hat.

Durch die US 2004/0139968 A1 ist ein Behälter, insbesondere Ampulle, gemäß dem Oberbegriff des Anspruchs 1. bekannt, bestehend aus mindestens einer Schicht an Kunststoffmaterial mit einer Abgabekammer, in der ein Abgabemedium bevorratbar ist, das über nur eine einzige Behälteröffnung abgebbar ist, die mittels eines lösbaren Verschlussteils verschliessbar ist, wobei die Abgabekammer eine weitere auf der gegenüberliegenden Seite der Abgabekammer angeordnete weitere Behälteröffnung aufweist, die jeweils mittels eines weiteren lösbaren Verschlussteils verschliessbar ist, nach dessen Entfernen die weitere Behälteröffnung in der Art eines "drain vent" ausgebildet ausschliesslich dem Belüften der Abgabekammer dient, um dergestalt eine Medienentnahme über die eine Behälteröffnung sicherstellen zu können.

Die bekannte Behälterlösung findet insbesondere Verwendung in sog. Aerosol-Abgabegeräten und beidseits des Behälterkörpers sind auf gegenüberliegenden Seiten in der Art von Einführschienen Längsstege mit unterschiedlichen Dicken vorgesehen, die eine definierte Positionierung des Abgabebehälters im Aerosolgerät ermöglichen, um dergestalt einen funktionssicheren Betrieb sicher zu stellen.

Durch die DE 35 25 098 A1 ist ein gattungsgemässer Behälter, insbesondere in Form einer balgförmigen Ampulle, bekannt, bestehend aus mindestens einer Schicht an Kunststoffmaterial mit einer Abgabekammer, in der ein Abgabemedium bevorratbar ist, das über eine Behälteröffnung abgebbar ist, die mittels eines lösbaren Verschlussteils verschliessbar ist, wobei die Abgabekammer eine weitere Behälteröffnung aufweist, die mittels eines weiteren lösbaren Verschlussteils verschliessbar ist und wobei die jeweilige Behälteröffnung eine Füll- und/oder Entnahmestelle für das jeweilige Abgabemedium bildet, die am freien Ende eines Halsteils, das in die Abgabekammer übergeht, angeordnet ist. Auf den genannten Behälter ist ein vergleichbarer Balg-Behälter aufschraubbar und nach Entfernen von folienartigen Verschlussteilen kann das Abgabemedium des einen Behälters mit dem Abgabemedium des anderen Behälters durch Betätigen der Faltenbälge in axialer Richtung vermischt werden und nach Aufsetzen eines spritzenartigen Düsenteils ist das derart gemischte Abgabemedium, beispielsweise in Form eines Epoxyd-Harzklebers, über die Düse nach außen hin abgebbar. Die bekannte Lösung ist insbesondere bezogen auf die an den Halsteilen angebrachten Gewindestrecken, die die Füll- und/oder Entnahmeanschlüsse ausbilden, aufwendig in der Herstellung und auch im Hinblick auf die eingesetzten Folienverschlüsse, die von Hand abziehbar sind, nicht geeignet, sterilen Anforderungen gerecht zu werden.

Ausgehend von diesem Stand der Technik liegt der Erfindung die Aufgabe zugrunde, die bekannten Sehälterlösungen dahingehend weiter zu verbessern, dass sie die beschriebenen Nachteile nicht aufweisen, dass sie insbesondere sich herstelltechnisch günstig erzeugen lassen und im Hinblick auf ihre Anschlusssystematik nicht nur eine höhere Varianz aufweisen, sondern auch sterilen Abgabeerfordemissen Rechnung tragen. Eine dahingehende Aufgabe löst ein Behälter mit den Merkmalen des Patentanspruches 1 in seiner Gesamtheit.

Dadurch, dass gemäß dem kennzeichnenden Teil des Patentanspruches 1 beidseits des Behälters Versteifungsstege verlaufen, die zunächst Bestandteil des Verschlussteiles sind, wobei der Verchlussteil einen Mittelteil aufweist, dass nach Entfernen des Mittelteils die eine Behälteröffnung freigegeben ist, die eine der Füll- und/oder Entnahmestellen bildet, und dass die Versteifungsstege bei entferntem Mittelteil einen Gegenhalt für eine weitere Ampulle bilden, ist nicht nur eine Behälterlösung erreicht, mit der sich die Werkzeuge und mithin die Herstellkosten reduzieren lassen, sondern auch eine steril dichtende Anlage im Bereich der Füll- und/oder Entnahmestelle der einen 8e-hälteröffnung, wozu die beidseitig am Behälter verlaufenden Versteifungsstege mit beitragen, die einen entsprechenden Gegenhalt bilden zu der Aufspreizbewegung betreffend das zugeordnete Halsteil, sobald ein Eingriff eines männlichen Eingriffteils der Ampulle für einen Medienentnahmevorgang in das Halsteil erfolgt.

Auch läßt sich der erfindungsgemäße Behälter mit seinen verschiedenen Arten an Behälteröffnungen mit vorhandenen Entnahmesystemen vor Ort koppeln, indem man einen jeweils bereits vorhandenen Füll- und/oder Entnahmeanschluss eben mit einer der vorhandenen Arten an Behälteröffnungen passend koppelt, die insoweit medizinischen Normstandards genügen.

Vorzugsweise ist dabei vorgesehen, dass verschiedene Arten an Behälteröffnungen voneinander verschiedene Halsgestaltungen aufweisen zum Anschluss verschiedener Füll- und/oder EntnahmeansChlüsse. Sofern vorzugsweise dabei die eine Art an Behälteröffnungen ein Halsteil aufweist, das sich zu seinem freien Ende hin mit seiner Außenmantelfläche konisch verjüngt oder zylindrisch ist und wobei die andere Art an Behälteröffnungen ein weiteres Halsteil aufweist, das sich zu seinem freien Ende hin mit seiner Innenmantelfläche konisch erweitert oder zylindrisch ist, sind für einen Behälter sowohl genormte männliche als auch weibliche Anschlussstellen konzipiert, die man in der Fachsprache auch mit Luer-Lock bezeichnet.

Bei einer besonders bevorzugten Ausführungsform des erfindungsgemäßen Behälters weisen die Abgabekammer und/oder das jeweilige Halsteil mindestens ein Rastmittel auf für den Angriff mindestens eines korrespondierenden Rastmittels des jeweiligen Füll- und/oder Entnahmeanschlusses. Neben der sicheren Festlegemöglichkeit des jeweiligen Anschlusses an den Behälter ist darüber hinaus in redundanter Weise eine Plausibilitätskontrolle in dem Sinne möglich, dass ein aufgebrachter Anschluss stimmig zu der zuordenbaren Abgabeöffnung ist und zwar jedenfalls dann, wenn die korrespondierenden Rastmittel miteinander sinnfällig zusammenwirken.

Im Folgenden wird der erfindungsgemäße Behälter anhand eines Ausführungsbeispieles nach der Zeichnung näher erläutert. Dabei zeigen in prinzipieller und nicht maßstäblicher Darstellung die
- Fig. 1: eine Vorderansicht auf einen Ampullenblock mit insgesamt vier Einzelbehältern,
- Fig. 2: teilweise eine Seitenansicht auf einen Einzelbehälter nach der Fig. 1,
- Fig. 3: einen Ausschnitt auf eine Behälterverbindung, bei der das Kopf- teil eines Behälters über eine Luer-Lock-Verbindung mit dem Fußteil eines anderen Behälters verbunden ist.

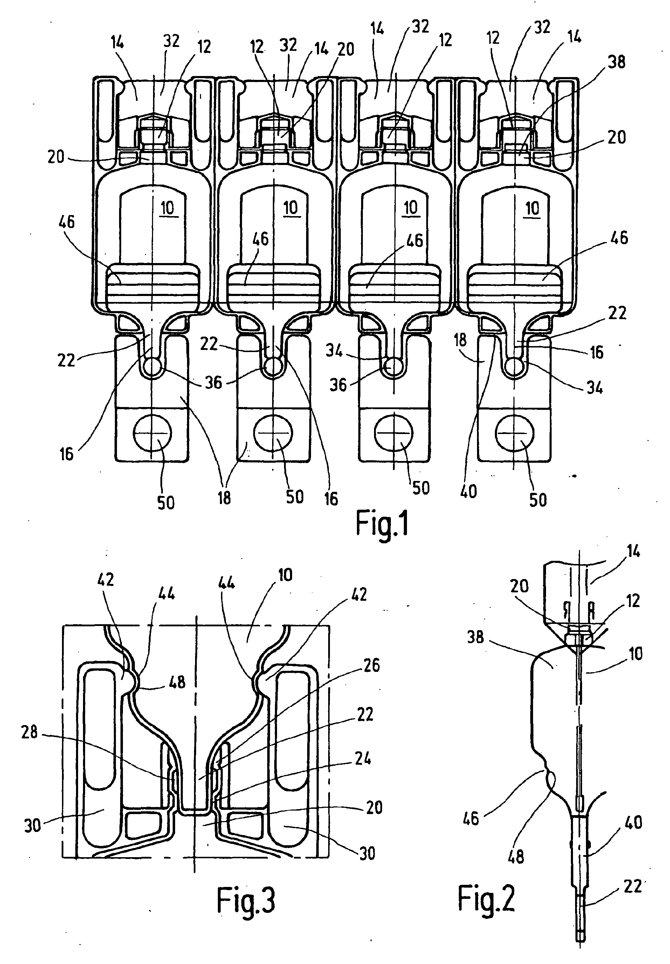

Der in Fig. 1 dargestellte Ampullenblock ist nur unvollständig dargestellt und zeigt insgesamt vier Behälter, wobei sich die Behälterreihe nach beiden Seiten hin entsprechend fortsetzen kann. Die einzelnen Behälter sind im so genannten Blasformverfahren oder mittels Vakuum aus Kunststoffmaterialien geformt, die hierfür entsprechend geeignet sind. Ferner besteht die Möglichkeit, die Behälter nach dem bottelpack^{®}-Verfahren herzustellen, bei dem in einer Arbeitsstation die Behälter blasgeformt, steril befüllt und verschlossen werden. Bereits während der gleichzeitig erfolgenden Verformung sowohl des einen als auch des benachbarten Behälters wird zwischen dem Körper der beiden Behälter eine diese zusammenhaltende Trennzone hergestellt, welche zunächst beide benachbarten Behälter miteinander verbunden hält, aber dadurch getrennt werden kann, dass man den einen Behälter relativ zu dem anderen Behälter um diese Trennzone bewegt.

Jeder Behälter weist eine Abgabekammer 10 auf, in der ein Abgabemedium bevorratbar ist. Dabei können die einzelnen Behälter eines Ampullenblokkes ein und dasselbe Abgabemedium aufweisen oder voneinander vierschiedene, insbesondere miteinander mischbare und des weiteren ist möglich, die Abgabekammer 10 leer zu belassen oder darin ein gasförmige Medium zu bevorraten, das dann gegebenenfalls mit Abgabemedien benachbarter Behälter entsprechend wechselwirkt. Das Abgabemedium ist über eine Behälteröffnung 12 abgebbar, die jeweils mittels eines lösbaren Verschlussteiles 14 verschließbar ist. Ferner weist die jeweilige Abgabekammer 10 mindestens eine weitere Behälteröffnung 16 auf, die jeweils mittels eines weiteren lösbaren Verschlussteiles 18 verschließbar ist.

Die jeweilige Behälteröffnung 12, 16 bildet eine Füll- und/oder Entnahmestelle für das jeweilige Abgabemedium aus, die am freien Ende eines Halsteiles 20 oder 22, das in die Abgabekammer 10 übergeht, angeordnet ist, wobei die verschiedenen Arten an Behälteröffnungen 12, 16 voneinander verschiedene Halsgestaltungen 20, 22 aufweisen zum Anschluss verschiedener Füll- und/oder Entnahmeanschlüsse, insbesondere in Form der korrespondierenden Halsteile 22 bzw. 20 eines weiteren Behälters.

So weist die eine Art an Behälteröffnungen 16 ein Halsteil 22 auf, das sich zu seinem freien Ende hin mit seiner Außenmantelfläche konisch verjüngt, wobei die andere Art an Behälteröffnungen 12 ein weiteres Halsteil 20 aufweist, das im Wesentlichen zylindrisch ausgebildet ist; sich zu seinem freien Ende hin aber auch mit seiner Innenmantelfläche konisch erweitern kann. Insbesondere bei einer zylindrischen Ausgestaltung ist eine entsprechende Kalibrierung vorgesehen, um einen guten Passsitz mit dem einzubringenden Konus, beispielsweise in Form des Halsteiles 22, sicherzustellen.

Die dahingehende Verbindungssituation ist entsprechend in der Fig. 3 dargestellt, wobei das Halsteil 20 des darunter liegenden Behälters eine erste zylindrische Passfläche 24 aufweist und eine weitere zweite darüber liegende Passfläche 26. Zwischen den beiden Passflächen 24 und 26 ist innerhalb des Halsteiles 20 ein Hohlraum 28 vorhanden, der zusammen mit der konischen Außerirnantelfläche des eindringenden Halsteiles 22 sicherstellt, dass eine steril dichtende Anlage über die Passflächen 24, 26 zustande kommt. Für eine dahingehend passgenaue Anlage dienen auch die beidseitig verlaufenden Versteifungsstege 30, die einen entsprechenden Gegenhalt bilden zu der Aufspreizbewegung betreffend das Halsteil 20. Die dahingehenden Versteifungsstege 30 sind zunächst Bestandteil des Verschlussteiles 14, wobei nach Entfernung des Mittelteiles 32 des Verschlussteils 14 die Behälteröffnung 12 für den Eingriff des männlichen Eingriffteiles 22 freigegeben ist. Die Verschlussteile 14, 18 sind somit zumindest teilweise in der Art eines auf diesem. Gebiet üblichen Knebelverschlusses ausgebildet, nach dessen Entfernen von einer vorgegebenen geschwächten Trennstelle eine Entnahmeöffnung bei dahingehenden Ampullenerzeugnissen freigebbar ist. Bei dem weiteren unteren Verschlussteil 18 ist die dahingehende Trennstelle 34 von einer Verschlusskugel 36 als Teil des weiteren Verschlussteiles 18 lösbar verschlossen. Gemäß dem gezeigten Ausführungsbeispiel sind also die jeweils verschiedenen Arten an Behälteröffnungen 12, 16 auf einander abgewandten Endseiten 38, 40 (Kopf- und Fußseite) angeordnet.

Des weiteren ist erfindungsgemäß vorgesehen, dass in radialer Quererweiterung zu dem Halsteil 20 an der Kopfseite des Behälters und in obiger Verlängerung des jeweiligen Versteifungssteges 30 ein Rastmittel 42 in Form einer vorspringenden Rastnase vorhanden ist, das in eine korrespondierende Ausnehmung als weiteres Rastmittel 44 an der Unterseite der Abgabekammer des anderen Behälters lösbar verrastbar ist. Hierfür ist die Ausnehmung 44 als konkave Vertiefung ausgebildet, in die die Rastnase des Rastmittels 42 konvex vorspringend in elastisch nachgiebiger Form einspringen kann. Für den dahingehenden Angriff ist, wie dies die Darstellung nach der Fig. 1 zeigt, am unteren Endbereich der Abgabekammer 10 diese flächig mit einer Riffelung 46 versehen, wobei eine dazwischenliegende Eingriffsnut 48 die Ausnehmung 44 bildet. Bei einer nicht näher dargestellten Ausführungsform besteht aber auch die Möglichkeit, auf dahingehende Rastmittel zu verzichten und die Verbindung nur über die übliche Luer-Lock-Systematik herzustellen.

Anstelle des gezeigten Ineinandergreifens zweier Behälter gemäß der Darstellung nach der Fig. 3 besteht aber auch die Möglichkeit, den jeweiligen Behälter mit einem externen Anschluss zu koppeln, um dergestalt einen Entnahmevorgang zu veranlassen. Aufgrund der beiden unterschiedlichen Entnahmesysteme an der Kopf- und an der Fußseite eines jeden Behälters erhöht sich die Entnahmemöglichkeit bezogen auf die hierfür vorgesehenen medizinisch standardisierten Anschlussstellen. Des weiteren kann der Behälter in mindestens einer Richtung Festlegeöffnungen 50 aufweisen, beispielsweise zum Anbringen von Produktinformationen, Patientendaten oder dergleichen mehr. Auch könnte dergestalt im Sinne einer Tropflösung über die Festlegeöffnung 50 der jeweilige Behälter an hierfür vorgesehenen Einrichtungen (nicht dargestellt) aufgehängt werden. Ferner besteht die Möglichkeit, in Reihe hintereinander auch mehr als zwei Behälter miteinander zu koppeln, sodass in größerem Rahmen unterschiedlichste Mischungen bereitgestellt werden können.

Zum Herstellen des Behälters kann auch ein so genanntes bottelpack^{®}-Coextrusionsverfahren dienen, bei dem das Kunststoffmaterial schlauchförmig extrudiert und zum Formen des jeweiligen Behälters mittels Differenzdruckes (Blasformen) an die Innenwände eines Formwerkzeuges angelegt wird, wobei der jeweilige Behälter über mindestens eine seiner Behälteröffnungen 12, 16 mit einer Fülleinrichtung mit dem jeweiligen Abgabemedium befüllt wird und die Behälteröffnung 12, 16 durch Verschließen steril geschlossen wird. Für das Extrudieren können dann verschiedene Kunststoffmaterialien im Coextrusionsverfahren eingesetzt werden, bei dem der jeweilige Behälter zumindest teilweise aus mehreren Schichten an Kunststoffmaterialien aufgebaut wird, wobei vorzugsweise mindestens eine der Schichten als Sperrschicht eingesetzt wird. Dabei können die einzelnen Schichten aus unterschiedlichen Kunststoffmaterialien gebildet werden, insbesondere Polyolefin, Polyamid, Polypropylen, Low-Density-Polyethylen, Copolymeren sowie Ethylen-Vinylalkohol-Copolymer.

## Patentansprüche

1. Behälter, insbesondere Ampulle, bestehend aus mindestens einer Schicht an Kunststoffmaterial, mit einer Abgabekammer (10), in der ein Abgabemedium bevorratbar ist, das über eine Behälteröffnung (12) abgebbar ist, die mittels eines lösbaren Verschlussteils (14) verschliessbar ist, wobei die Abgabekammer (10) mindestens eine weitere Behälteröffnung (16) aufweist, die jeweils mittels eines weiteren lösbaren Verschlussteils (18) verschliessbar ist, und wobei die jeweilige Behälteröffnung (12,16) eine Füll- und/oder Entnahmestelle für das jeweilige Abgabemedium bildet, die am freien Ende eines Halsteils (20,22), das in die Abgabekammer (10) übergeht, angeordnet ist, **dadurch gekennzeichnet, dass** beidseits des Behälters Versteifungsstege (30) verlaufen, die zunächst Bestandteil des Verschlussteiles (14) sind, wobei der Verschlussteil (14) einen Mittelteil (32) aufweist, dass nach Entfernen des Mittelteils (32) die eine Behälteröffnung (12) freigegeben ist, die eine der Füll- und/oder Entnahmestellen bildet, und dass die Versteifungsstege (30) bei entferntem Mittelteil (32) einen Gegenhalt für eine weitere Ampulle bilden.

2. Behälter nach Anspruch 1, **dadurch gekennzeichnet, dass** die verschiedenen Arten an Behälteröffnungen (12,16) voneinander verschiedene Halsgestaltungen (20,22) aufweisen zum Anschluss verschiedener Füll- und/oder Entnahmeanschlüsse.

3. Behälter nach Anspruch 2, **dadurch gekennzeichnet, dass** die eine Art an Behälteröffnungen (16) ein Halsteil (22) aufweist, das sich zu seinem freien Ende hin mit seiner Aussenmantelfläche konisch verjüngt oder zylindrisch ist und dass die andere Art an Behälteröffnungen (12) ein weiteres Halsteil (20) aufweist, das sich zu seinem freien Ende hin mit seiner Innenmantelfläche konisch erweitert oder zylindrisch ist.

4. Behälter nach einem der Ansprüche 1 bis 3, **dadurch gekennzeichnet, dass** die Abgabekammer (10) an ihren einander abgewandten Endseiten (38, 40) (Kopf- und Fussseite) und koaxial zu ihrer Längsachse jeweils eine Art an Behälteröffnungen (12, 16) aufweist.

5. Behälter nach einem der Ansprüche 1 bis 4, **dadurch gekennzeichnet, dass** die Verschlussteile (14, 18) in der Art eines Knebelverschlusses ausgebildet sind.

6. Behälter nach einem der Ansprüche 2 bis 5, **dadurch gekennzeichnet, dass** die Abgabekammer (10) und/oder das jeweilige Halsteil (20, 22) mindestens ein Rastmittel (42, 44) aufweisen für den Angriff mindestens eines korrespondierenden Rastmittels (42, 44) des jeweiligen Füll- und/oder Entnahmeanschlusses.

7. Behälter nach einem der Ansprüche 1 bis 6, **dadurch gekennzeichnet, dass** er in Richtung mindestens einer der jeweiligen Behälteröffnungen (12, 16) Festlegeöffnungen (50) aufweist zum Anbringen von Produktinformationen, Patientendaten etc..

8. Behälter nach einem der Ansprüche 1 bis 6, **dadurch gekennzeichnet, dass** er mehrere Schichten an Kunststoffmaterialien aufweist, die über ein Coextrusionsverfahren herstellbar sind.

## Claims

1. A container, in particular an ampoule, comprising at least one layer of plastic material, having a discharge chamber (10) in which a discharge medium can be stored and which can be discharged via a container opening (12) which can be closed by means of a releaseable closing part (14), the discharge chamber (10) having at least one further container opening (16) which can respectively be closed by means of a further releaseable closing part (18), and the respective container opening (12) forming a filling and/or removal point for the respective discharge medium which is disposed on the free end of a neck part (20, 22) which passes into the discharge chamber (10), **characterised in that** there are extending on both sides of the container reinforcement bars (30) which are initially a component part of the closing part (14), the closing part (14) having a central part (32), that after removing the central part (32) the one container opening (12), which forms one of the filling and/or removal points, is cleared, and that when the central part (32) is removed the reinforcement bars (30) form a retainer for a further ampoule.

2. The container according to Claim 1, **characterised in that** the different types of container openings (12, 16) have different neck designs (20, 22) for the attachment of different filling and/or removal attachments.

3. The container according to Claim 2, **characterised in that** the one type of container opening (16) has a neck part (22) which tapers conically towards its free end with its outer shell, or is cylindrical, and that the other type of container opening (12) has a further neck part (20) which extends conically towards its free end with its inner shell, or is cylindrical.

4. The container according to any of Claims 1 to 3, **characterised in that** the discharge chamber (10) has respectively on its end sides (38, 40) (head and foot side) facing away from one another and coaxially to its longitudinal axis a type of container opening (12, 16).

5. The container according to any of Claims 1 to 4, **characterised in that** the closing parts (14, 18) are designed in the manner of a toggle fastener.

6. The container according to any of Claims 2 to 5, **characterised in that** the discharge chamber (10) and/or the respective neck part (20, 22) have at least one engagement means (42, 44) for the engagement of at least one corresponding engagement means (42, 44) of the respective filling and/or removal attachment.

7. The container according to any of Claims 1 to 6, **characterised in that** it has in the direction of at least one of the respective container openings (12, 16) fixing openings (50) for affixing product information, patient data etc..

8. The container according to any of Claims 1 to 6, **characterised in that** it has a number of layers of plastic materials which can be produced using a coextrusion method.

## Revendications

1. Récipient, notamment une ampoule, constitué d'au moins une couche de matière plastique, comprenant une chambre (10) de dégagement, dans laquelle peut être mis en réserve un milieu de dégagement qui peut être dégagé par une ouverture (12) du récipient, laquelle peut être fermée au moyen d'une partie (14) de fermeture amovible, la chambre (10) de dégagement ayant au moins une autre ouverture (16) de récipient, qui peut être fermée respectivement au moyen d'une autre partie (18) de fermeture amovible et dans lequel l'ouverture (12, 16) respective du récipient forme un point de remplissage et/ou de prélèvement du milieu de dégagement respectif disposé à l'extrémité libre d'une partie (20, 22) de col se transformant en la chambre (10) de dégagement, **caractérisé en ce que** des deux côtés du récipient s'étendent des nervures (30) de raidissement, qui font au moins partie de la partie (14) de fermeture, la partie (14) de fermeture ayant une partie (32) médiane, **en ce qu'**après avoir enlevé la partie (32) médiane ladite une ouverture (12) du récipient, qui forme l'un des points de remplissage et/ou de prélèvement, est dégagée et **en ce que** les nervures (30) de raidissement forment, lorsque la partie (32) médiane est enlevée, un contre-appui pour une ampoule suivante.

2. Récipient suivant la revendication 1, **caractérisé en ce que** les divers types d'ouvertures (12, 16) de récipient ont des conformations (20, 22) de col qui diffèrent l'une de l'autre pour le raccordement de raccords de remplissage et/ou de prélèvement différents.

3. Récipient suivant la revendication 2, **caractérisé en ce qu'**un type d'ouverture (16) de récipient a une partie (22) de col, qui se rétrécit coniquement par sa surface latérale extérieure jusqu'à son extrémité libre ou qui est cylindrique, et **en ce que** l'autre type d'ouverture (12) de récipient a une autre partie (20) de col, qui s'élargit coniquement par sa surface latérale intérieure vers son extrémité libre ou est cylindrique.

4. Récipient suivant l'une des revendications 1 à 3, **caractérisé en ce que** la chambre (10) de dégagement a, sur ses côtés (38, 40) d'extrémité éloignés l'un de l'autre (côté de tête et côté de queue) et coaxialement à son axe longitudinal, respectivement un type d'ouvertures (12, 16) de récipient.

5. Récipient suivant l'une des revendications 1 à 4, **caractérisé en ce que** les parties (14, 18) de fermeture sont constituées à la manière d'une fermeture à manette.

6. Récipient suivant l'une des revendications 2 à 5, **caractérisé en ce que** la chambre (10) de dégagement et/ou la partie (20, 22) de col correspondante ont au moins un moyen (42, 44) d'encliquetage pour l'attaque d'au moins un moyen (42, 44) d'encliquetage correspondant du raccord respectif de remplissage et/ou de prélèvement.

7. Récipient suivant l'une des revendications 1 à 6, **caractérisé en ce qu'**il a, dans la direction d'au moins l'une des ouvertures (12, 16) respectives de récipient, des ouvertures (50) de fixation pour mettre des informations de produit, des données de patient, etc.

8. Récipient suivant l'une des revendications 1 à 6, **caractérisé en ce qu'**il a plusieurs couches de matières plastiques, qui peuvent être produites par un procédé de coextrusion.
